# EUROPEAN PATENT APPLICATION

(11) **EP 4 410 280 A1**
(43) Date of publication of application: **07.08.2024**
(21) Application number: 24155382.5
(22) Date of filing: 01.02.2024
(51) Int. Cl.: A61K 9/20, A61K 9/28, A61K 31/155, A61K 31/7048

(54) **FILM COATING TABLETS OF EMPAGLIFLOZIN AND METFORMIN HYDROCHLORIDE**

(30) Priority: 02.02.2023 TR 202301202
(71) Applicant: SANOVEL ILAC SANAYI VE TICARET A.S., Istanbul 34460 (TR)
(72) Inventor: SUNEL, Fatih, Istanbul (TR); PEHLIVAN AKALIN, Nur, Istanbul (TR); KIVANC, Furkan, Istanbul (TR); GULER, Tolga, Istanbul (TR)
(74) Representative: Sevinç, Erkan

(57) **Abstract**

The present invention relates to a film coated tablet comprising empagliflozin and metformin hydrochloride wherein comprising at least one disintegrant which is croscarmellose sodium, sodium starch glycollate, sodium alginate, starch, magnesium aluminum silicate or a mixture thereof. The present invention also relates to a simple, rapid, cost effective, time-saving and industrially convenient method of preparing the tablet.

## Description

### Field of the Invention

The present invention relates to a film coated tablet comprising empagliflozin and metformin hydrochloride wherein comprising at least one disintegrant which is croscarmellose sodium, sodium starch glycollate, sodium alginate, starch, magnesium aluminum silicate or a mixture thereof. The present invention also relates to a simple, rapid, cost effective, time-saving and industrially convenient method of preparing the tablet.

### Background of the Invention

Diabetes mellitus is a group of disorders of carbohydrate metabolism in which the action of insulin is diminished or absent through altered secretion, decreased insulin activity or a combination of both factors. There are two main types of diabetes; Type 1 and Type 2: Type 1 diabetes occurs because the insulin-producing cells of the pancreas (beta cells) are damaged. In Type 1 diabetes, the pancreas makes little or no insulin, so sugar cannot get into the body's cells for use as energy. People with Type 1 diabetes must use insulin injections to control their blood glucose. In Type 2 diabetes, the pancreas makes insulin, but it either doesn't produce enough, or the insulin does not work properly. This diabetes occurs most often in people who are over 40 years old and overweighed. Type 2 diabetes may sometimes be controlled with a combination of diet, weight management, and exercise. However, treatment also may include oral glucose-lowering medications or insulin injections.

Metformin is antidiabetics having an orally-administrated biguanide structure. Metformin hydrochloride is a white to off-white crystalline compound and it is freely soluble in water and practically insoluble in acetone, ether and chloroform. Oral doses of metformin are generally recommended in the range of 500 to 2500 mg a day and a single dose may vary from 500 to 850 mg. It is used singly or in combination with sulfonylureas, alpha-glucosidase inhibitors, or insulin.

The chemical name of metformin hydrochloride is 1,1-dimethylbiguanide hydrochloride, has the following chemical structure of Formula I.

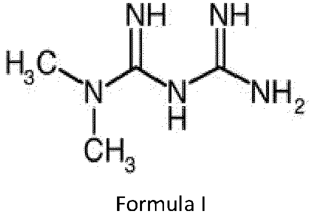

Empagliflozin is a known SGLT2 inhibitor that is described for the treatment or improvement in glycemic control in patients with type 2 diabetes mellitus. The chemical name of empagliflozin is 1 - chloro-4-(3-D-glucopyranos-1 -yl)- 2-[4-((S)-tetrahydrofuran-3-yloxy)-benzyl]-benzene and its chemical structure is shown in the Formula II.

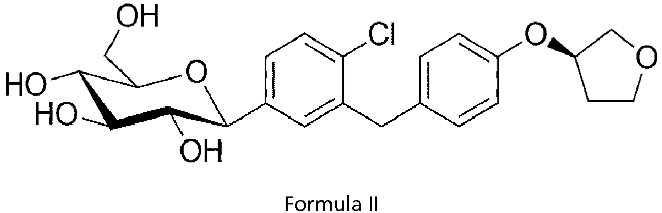

Combination product of empagliflozin and metformin hydrochloride is marketed under the trademark Synjardy^{®}. The combination is to help control blood glucose in people with T2D. Empagliflozin, a sodium glucose co-transporter-2 (SGLT2) inhibitor, removes excess glucose through the urine by blocking glucose re-absorption in the kidney.

Active ingredients have some disadvantages in the formulation and process. The main problem encountered when preparing formulations comprising empagliflozin is low solubility, leading to difficulties with disintegration and dissolution times. Furthermore, metformin is a very poorly compressible active substance and metformin presents in high amounts in a composition. This causes some problems for examples; compressibility, homogeneity, flowability and dissolution profile.

WO2011039337 (A1) application discloses pharmaceutical compositions comprising fixed dose combinations of a SGLT-2 inhibitor drug and a partner drug, processes for the preparation thereof, and their use to treat certain diseases.

CN104586834 (A) application discloses a pharmaceutical composition of empagliflozin and metformin, a preparation method and application thereof. The composition comprises the following components: i.) empagliflozin; ii.) metformin hydrochloride; and one or more fillers; one or more adhesives; one or more flow aids and one or more lubricants.

In the prior art, there are also several patents which disclose empagliflozin and metformin hydrochloride in oral pharmaceutical dosage forms. However, because of the dissolution problem of empagliflozin, and the poorly compressible of metformin, an effective formulation and method has not been disclosed.

There still remains a need in the art to provide an improved a film coated tablet comprising empagliflozin and metformin hydrochloride having high solubility, excellent pharmacotechnical properties and accordingly a high bioavailability and a long-term stability which is also obtained by using an effective process.

### Detailed Description of the Invention

The main object of the present invention is to provide a film coated tablet comprising empagliflozin and metformin hydrochloride with having the desired level of dissolution rate and excellent physicochemical properties, such as flowability, compressibility, homogeneity, and content uniformity which overcomes the above-described problems in the prior art and have additive advantages over them.

Another object of the present invention is to provide a film coated tablet comprising empagliflozin and metformin hydrochloride with having high stability.

Another object of the present invention is to provide a process for preparing a film coated tablet comprising empagliflozin and metformin hydrochloride. The process is a simple, rapid, cost effective, time-saving, and industrially convenient method.

The main problem encountered when preparing formulations comprising empagliflozin is low solubility, leading to difficulties with disintegration and dissolution times. Furthermore, metformin is a very poorly compressible active substance and metformin presents in high amounts in a composition. This causes some problems for examples; compressibility, homogeneity, flowability and dissolution profile. Therefore, the excipients and process steps used are very important. We found that the selected disintegrant excipient eliminated the described above problems.

According to an embodiment of the present invention, a film coated tablet comprises metformin hydrochloride and empagliflozin wherein comprising at least one disintegrant which is croscarmellose sodium, crospovidone, sodium starch glycollate, sodium alginate, starch, magnesium aluminum silicate or a mixture thereof. The film coated tablet is obtained which provides the desired dissolution profile. Also, it has been surprisingly found that the selected disintegrant provides good compressibility of the tablet. This choice of disintegrant eliminates the disadvantages of both active agents and provides an effective tablet formulation.

According to an embodiment of the present invention, preferably, the disintegrant is croscarmellose sodium.

According to an embodiment of the present invention, the amount of disintegrant is 1.0% to 15.0% by weight in the total composition. Preferably, the amount of disintegrant is 2.0% to 7.0% by weight in the total composition.

According to an embodiment of the present invention, a film coated tablet comprises 70.0% to 90.0% of metformin hydrochloride and 0.1% to 5.0% of empagliflozin by weight in the total composition.

According to an embodiment of the present invention, the film coated tablet further comprises at least one diluent, at least one binder and at least one glidant/lubricant.

Encountered while developing formulations is the flowability-problem and homogeneity of metformin HCl, which makes the production difficult. It has been observed that this problem is overcome by using at least one diluent.

Suitable diluents are selected from the group comprising lactose monohydrate, corn starch, microcrystalline cellulose, lactose, anhydrous lactose, starch, mannitol, calcium hydrogen phosphate dihydrate, dicalcium hydrogen phosphate anhydrate, calcium phosphate trihydrate, neutral pellets, magnesium carbonate, magnesium oxide, maltodextrin, maltose, medium chain triglycerides or mixtures thereof.

According to an embodiment of the present invention, the diluent is lactose monohydrate. The filler provides flowability and homogeneity of the powder mixture.

According to an embodiment of the present invention, the amount of diluents is 1.0% to 15.0% by weight in the total composition. Preferably, the amount of diluents is 1.0% to 7.0% by weight in the total composition.

Suitable binders are selected from the group comprising hydroxypropyl cellulose, hydroxypropyl methylcellulose, pregelatinized starch, polyvinylpyrrolidone, carboxymethylcellulose sodium, cellulose acetate phthalate, chitosan, dextrose, ethylcellulose, glyceryl behenate, hydroxyethyl cellulose, hydroxyethylmethyl cellulose, hydroxypropyl starch, magnesium aluminum silicate, polyethylene oxide, polymethacrylates, polyoxyethilene-alkyl ethers or mixtures thereof.

According to an embodiment of the present invention, the amount of binders is 2.0% to 15.0% by weight in the total composition. Preferably, the amount of binders is 2.0% to 10.0% by weight in the total composition.

According to an embodiment of the present invention, the binder is hydroxypropyl cellulose. Especially, it is hydroxypropyl cellulose LF.

Suitable glidants/lubricants are selected from the group comprising anhydrous colloidal silicon dioxide, magnesium stearate, sodium stearyl fumarate, magnesium oxide, starch, silicone dioxide, talc, polyethylene glycol, stearic acid, aluminum silicate, magnesium silicate, colloidal silica or mixtures thereof.

According to an embodiment of the present invention, the glidant/lubricant is anhydrous colloidal silicon dioxide or magnesium stearate or mixtures thereof. These excipients provide the flowability of the powder mixture.

According to an embodiment of the present invention, the amount of glidants/lubricants is 0.2% to 4.0% by weight in the total composition. Preferably, the amount of glidants/lubricants is 0.5% to 2.5% by weight in the total composition.

According to an embodiment of the present invention, the film coated tablet is coated with at least one film coating agent. Especially, HPMC-based coating is used.

Suitable film coating agents are selected from the group comprising polymethacrylates, hydroxypropyl methylcellulose, lactose monohydrate, talc, hydroxypropyl cellulose, polyvinyl alcohol (PVA), polyethylene glycol (PEG), glycerin, polyvinyl alcohol-polyethylene glycol copolymers (Kollicoat^{®} IR), ethylcellulose dispersions (Surelease^{®}), polyvinylprolidone, polyvinylprolidone-vinyl acetate copolymer (PVP-VA), iron oxide yellow, iron oxides, all kinds of Opadry^{®}, pigments, dyes, titanium dioxide, coloring agent or mixtures thereof.

According to an embodiment of the present invention, the film coated tablet comprises;
a) Metformin HCl
b) Empagliflozin
c) Croscarmellose sodium
d) Lactose monohydrate
e) Hydroxypropyl Cellulose.

According to an embodiment of the present invention, the film coated tablet comprises;
a) Metformin HCl
b) Empagliflozin
c) Croscarmellose sodium
d) Lactose monohydrate
e) Hydroxypropyl Cellulose LF
f) Anhydrous colloidal silicon dioxide
g) Magnesium stearate

The film coated tablet of the present invention is prepared wet granulation.

According to one embodiment of the present invention, a process for the preparation of the film coated tablet comprises the following steps:
a) Sieving metformin HCl,
b) Mixing Metformin HCl, empagliflozin, lactose monohydrate and hydroxypropyl Cellulose,
c) Granulating the mixture with pure water,
d) Drying the wet granules and sieving,
e) Sieving anhydrous colloidal silicon dioxide,
f) Adding anhydrous colloidal silicon dioxide, magnesium stearate and croscarmellose sodium and then adding the mixture at step (d) and mixing,
g) Compressing the mixture into the tablet,
h) Coating the tablets with film coating agent.

According to this embodiment of the present invention, a solvent is used at wet granulation.

Suitable solvents are selected from the group comprising pure water, dichloromethane, 0.1N HCl, methanol, ethanol, isopropyl alcohol, benzyl alcohol, propylene glycol, polyethylene glycol, cyclomethicone or mixtures thereof. Preferably, the solvent is water.

### Example 1: The tablet formulation

| **Ingredients** | **% by weight** |
|---|---|
| Metformin HCl | 70.0 - 90.0 |
| Empagliflozin | 0.1 - 5.0 |
| Croscarmellose sodium | 1.0-15.0 |
| Lactose monohydrate | 1.0 - 15.0 |
| Hydroxypropyl Cellulose | 2.0 - 15.0 |
| Anhydrous colloidal silicon dioxide | 0.1 - 2.0 |
| Magnesium stearate | 0.1 - 2.0 |
| **TOTAL** | **100** |
| Coating | |

### Example 2: The tablet formulation

| **Ingredients** | **% by weight** | **% by weight** |
|---|---|---|
| Metformin HCl | 83.3 | 83.3 |
| Empagliflozin | 2.1 | 0.4 |
| Croscarmellose sodium | 5.0 | 5.0 |
| Lactose monohydrate | 1.8 | 3.4 |
| Hydroxypropyl Cellulose (L fine) | 6.7 | 6.7 |
| Anhydrous colloidal silicon dioxide | 0.3 | 0.3 |
| Magnesium stearate | 0.8 | 0.8 |
| **TOTAL** | **100** | **100** |
| Coating | | |

A process for example 1 or 2;
a) Sieving metformin HCl through 2 mm,
b) Mixing Metformin HCl, empagliflozin, lactose monohydrate and hydroxypropyl Cellulose
c) Granulating the mixture with a solvent (for example; pure water)
d) Drying the wet granules at Fluid Bed Dryer and sieving through 1.0 mm,
e) Sieving anhydrous colloidal silicon dioxide through 0.85,
f) Adding anhydrous colloidal silicon dioxide, magnesium stearate and croscarmellose sodium and then adding the mixture at step (d) and mixing,
g) Compressing the mixture into the tablet,
h) Coating the tablets with film coating agent.

## Claims

1. A film coated tablet comprises metformin hydrochloride and empagliflozin wherein comprising at least one disintegrant which is croscarmellose sodium, sodium starch glycolate, crospovidone, sodium alginate, starch, magnesium aluminum silicate or a mixture thereof.

2. The film coated tablet according to claim 1, wherein the disintegrant is croscarmellose sodium.

3. The film coated tablet according to claim 1, wherein the amount of disintegrant is 1.0% to 15.0% by weight in the total composition.

4. The film coated tablet according to claim 1, wherein comprises 70.0% to 90.0% of metformin hydrochloride and 0.1% to 5.0% of empagliflozin by weight in the total composition.

5. The film coated tablet according to claim 1, wherein further comprises at least one diluent, at least one binder and at least one glidant/lubricant.

6. The film coated tablet according to claim 5, wherein diluents are selected from the group comprising lactose monohydrate, corn starch, microcrystalline cellulose, lactose, anhydrous lactose, starch, mannitol, calcium hydrogen phosphate dihydrate, dicalcium hydrogen phosphate anhydrate, calcium phosphate trihydrate, neutral pellets, magnesium carbonate, magnesium oxide, maltodextrin, maltose, medium chain triglycerides or mixtures thereof.

7. The film coated tablet according to claim 6, wherein the diluent is lactose monohydrate.

8. The film coated tablet according to claim 6 or 7, wherein the amount of diluents is 1.0% to 15.0% by weight in the total composition.

9. The film coated tablet according to claim 5, wherein binders are selected from the group comprising hydroxypropyl cellulose, hydroxypropyl methylcellulose, pregelatinized starch, polyvinylpyrrolidone, carboxymethylcellulose sodium, cellulose acetate phthalate, chitosan, dextrose, ethylcellulose, glyceryl behenate, hydroxyethyl cellulose, hydroxyethylmethyl cellulose, hydroxypropyl starch, magnesium aluminum silicate, polyethylene oxide, polymethacrylates, polyoxyethilene-alkyl ethers or mixtures thereof.

10. The film coated tablet according to claim 9, wherein the amount of binders is 2.0% to 15.0% by weight in the total composition.

11. The film coated tablet according to claim 9, wherein the binder is hydroxypropyl cellulose. Especially, it is hydroxypropyl cellulose LF.

12. The film coated tablet according to claim 5, wherein glidants/lubricants are selected from the group comprising anhydrous colloidal silicon dioxide, magnesium stearate, sodium stearyl fumarate, magnesium oxide, starch, silicone dioxide, talc, polyethylene glycol, stearic acid, aluminum silicate, magnesium silicate, colloidal silica or mixtures thereof.

13. The film coated tablet according to claim 12, wherein the glidant/lubricant is anhydrous colloidal silicon dioxide or magnesium stearate or mixtures thereof.

14. The film coated tablet according to claim 5, wherein comprises;
a) Metformin HCl
b) Empagliflozin
c) Croscarmellose sodium
d) Lactose monohydrate
e) Hydroxypropyl Cellulose.
